# EUROPÄISCHE PATENTSCHRIFT

(11) **EP 2 509 518 B1**
(45) Veröffentlichungstag und Bekanntmachung des Hinweises auf die Patenterteilung: **27.04.2016**
(21) Anmeldenummer: 10795638.5
(22) Anmeldetag: 30.11.2010
(51) Int. Cl.: A61B 17/29

(54) **CHIRURGISCHES MANIPULATIONSINSTRUMENT**
SURGICAL MANIPULATION INSTRUMENT
INSTRUMENT DE MANIPULATION À USAGE CHIRURGICAL

(30) Priorität: 07.12.2009 DE 102009060987
(43) Veröffentlichungstag der Anmeldung: 17.10.2012
(73) Patentinhaber: Deutsches Zentrum für Luft- und Raumfahrt e.V., 51147 Köln (DE)
(72) Erfinder: SEIBOLD, Ulrich, Burnaby BC V3N 4Z4 (CA); LANTERMANN, Sophie, 81679 München (DE)
(74) Vertreter: von Kreisler Selting Werner - Partnerschaft von Patentanwälten und Rechtsanwälten mbB
(86) Internationale Anmeldenummer: PCT/EP2010/068506
(87) Internationale Veröffentlichungsnummer: WO 2011/069863

(56) Entgegenhaltungen:
- WO-A1-03/077769
- US-A1- 2003 055 409
- US-A1- 2008 021 278

## Beschreibung

Die Erfindung bezieht sich auf ein chirurgisches Manipulationsinstrument, mit einer Kupplung, die ein extrakorporales Antriebsteil mit einem teilweise intrakorporalen Manipulatorteil trennbar verbindet.

Chirurgische Instrumente, die nicht zum einmaligen Gebrauch, sondern zum mehrfachen Gebrauch konzipiert sind, müssen nach jedem Gebrauch sterilisiert werden. Die Sterilisierung kann mit Hilfe nicht-thermischer oder thermischer Verfahren durchgeführt werden. Im klinischen Alltag wird im wesentlichen mit thermischen Verfahren sterilisiert, insbesondere durch die sogenannte Autoklavierung. Bei der Autoklavierung wird das zu sterilisierende Instrument über einen bestimmten Zeitraum überspanntem Wasserdampf ausgesetzt, der alle zu sterilisierenden Oberflächen benetzen muss. Das zu sterilisierende Instrument ist dabei im Autoklaven Temperaturen von bis zu 156 °C und Drücken bis zu 2 bar über eine Dauer von bis zu 40 Min. ausgesetzt. Die Autoklavierung muss nach jedem Gebrauch des Instruments wiederholt werden, so dass im Laufe eines Instrumentenlebens bis zu mehrere Hundert Autoklavierungen durchlaufen werden können.

Seit den 1980er Jahren gewinnt die sogenannte minimal invasive Chirurgie immer mehr an Bedeutung. Hierbei werden lange, schlanke Manipulationsinstrumente durch nur kleine Öffnungen in der Oberhaut vorgeschoben. Das intrakorporale Operationsfeld wird mit Hilfe einer auf die gleiche Weise eingebrachten stabförmigen Kamera und eines extrakorporalen Monitors beobachtet. Die minimal invasive Chirurgie bietet insbesondere Vorteile für den Patienten, nämlich geringe Traumatisierung, kurze Rekonvaleszenzzeiten, geringere postoperative Schmerzen, geringeren Blutverlust, geringeres Infektionsrisiko, geringeres Risiko für Wundheilungsstörungen, bessere kosmetische Ergebnisse etc. Nachteile der minimal invasiven Chirurgie sind u.a. die eingeschränkte Bewegungsfreiheit der chirurgischen Instrumente. Durch die als feststehend anzusehende Durchtrittsstelle durch das Oberhaut- und Fettgewebe, die einen invarianten Punkt bildet, ergeben sich umgekehrte Bewegungsverhältnisse bzw. eine gestörte Hand-Auge-Koordination zum Monitorbild. Zwei Freiheitsgrade der Bewegung sind durch den invarianten Punkt gebunden, d.h. nicht jeder Punkt im Arbeitsraum kann mit beliebiger Orientierung des funktionalen Instrumentenendes erreicht werden.

Minimal invasive Manipulationsinstrumente, die intrakorporal zusätzliche Freiheitsgrade der Bewegung bieten, können zu einer erhöhten intrakorporalen Manipulabilität verhelfen und stellen damit eine wichtige Verbesserung für die minimal invasive Chirurgie dar. Die zusätzlichen Freiheitsgrade müssen zielgerichtet bewegt werden. Dies ist mit manueller Bedienung zwar möglich, jedoch erfordert dies großes Geschick und Übung. Ein robotergestützter telemanipulierter Ansatz, bei dem der Chirurg abseits des Patienten an einer ergonomisch geformten Konsole sitzt und mit Hilfe einer geeigneten Mensch-Maschine-Schnittstelle das chirurgische Manipulationsinstrument mit Hilfe des Monitors führt, ohne über die Kinematik und deren Aktuierung nachdenken zu müssen, ist daher sinnvoll. Das chirurgische Manipulationsinstrument wird dabei rechnergestützt aktuiert und führt die vom Chirurgen gewünschte Bewegung entsprechend aus.

Die Aktuatoren zum Antrieb des chirurgischen Manipulationsinstruments können in der Regel jedoch nicht autoklaviert werden. Daher ist eine Trennbarkeit des extrakorporalen Antriebsteiles von dem teilweise intrakorporalen Manipulatorteil erforderlich.

Das chirurgische Manipulationsinstrument wird daher zweiteilig und durch eine Kupplung trennbar in ein extrakorporales Antriebsteil und ein teilweise intrakorporales Manipulatorteil getrennt.

Aus US 6 491 707 A1 ist ein chirurgisches Manipulationsinstrument mit einer Kupplung zur Trennung des Antriebsteiles von dem Manipulatorteil bekannt. Hierbei werden durch die Antriebseinrichtung Betätigungselemente wie Wellen rotiert und über die Kupplungseinrichtung die Rotations- und Drehbewegung an Betätigungselemente des Manipulatorteils übertragen. Durch diese zweiten Betätigungselemente wird der Endeffektor des Manipulatorteils betätigt. Die Kupplungsteile weisen jeweils drehbare Kupplungskörper auf, die axiale Stifte bzw. Bohrungen aufweisen und axial miteinander verkuppelt bzw. voneinander getrennt werden. Da die drehbaren einander gegenüberstehenden Kupplungskörper nicht zusammen kuppelbar sind, wenn sie nicht genau zueinander ausgerichtet sind, muss beim Einkuppeln ein Suchlauf für alle Kupplungskörper-Paare durchgeführt werden. Die Kupplungskörper rotieren jeweils so lange bis eine Position gefunden ist, in der alle Kupplungskörper-Paare in einer zusammenführbaren Kupplungsposition stehen. Eine ähnliche Kupplung ist aus US 2001 003 1983 A1 bekannt. Die Kupplungsteile weisen hier halbzylindrisch geformte Kupplungskörper auf. Für einen leichtgängigen Kupplungsvorgang ist es erforderlich, ein gewisses Mindest-Spiel zwischen den Kupplungskörpern im zusammengekuppelten Zustand vorzusehen. Dieses Spiel wirkt sich jedoch beim Betrieb des Manipulatorinstruments nachteilig aus bzw. macht eine automatische Regelung ggf. sogar unmöglich.

In US 2008/0021278 A1 wird ein chirurgisches Manipulationsinstrument beschrieben, bestehend aus einem Aktuatorteil und einem durch eine Kupplung trennbaren Manipulatorteil mit Endeffektor. Dabei weist das Aktuatorteil im Bereich der Kupplung nach innen schwenkbare Kupplungsarme auf, die in die dafür vorgesehenen Hinterschnitt-Ausbuchtungen im Manipulatorteil eingreifen und damit die Kupplung verschließen. Gleichzeitig wird das erste axial bewegliche Betätigungselement des Aktuatorteils mit dem zweiten axial beweglichen Betätigungselement des Manipulatorteils verbunden, so dass eine Kraftübertragung vom Aktuator auf den Endeffektor möglich ist.

Ferner ist aus US 2009/0055409 A1 eine Vorrichtung zur Dispensierung von Flüssigkeiten für die minimal invasive Chirurgie bekannt, das eine austauschbare Auslassnadel besitzt. Das Kupplungselement zwischen Flüssigkeitseinlassvorrichtung und Auslassnadel umfasst dabei mehrere Kupplungsarme, die an ihren freien Enden nach innen gerichtete Ansätze aufweisen. Beim Zusammenstecken von Flüssigkeitseinlassvorrichtung und Auslassnadel werden die Kupplungsarme auseinander gedrückt bis diese in der Geschlossenstellung in die dafür vorgesehenen Hinterschnitt-Ausbuchtungen der Flüssigkeitseinlassvorrichtung einrasten.

Aufgabe der Erfindung ist es ein chirurgisches Manipulationsinstrument mit sowohl antriebsseitig als auch manipulatorseitig axial verschiebbaren Elementen zu schaffen, wobei die Betätigungselemente durch eine zuverlässige und einfache Kupplungseinrichtung miteinander verbunden sind.

Die Lösung der Aufgabe erfolgt erfindungsgemäß durch die Merkmale des Anspruchs 1.

Durch neuentwickelte axiale Bewegungen erzeugende Antriebseinrichtungen ist es möglich erste mit der Antriebseinrichtung verbundene Betätigungselemente wie Seile oder Stäbe nicht zu drehen sondern axial, d.h. in der Längsrichtung zu verschieben. Auch auf der Seite des Manipulatorteils ist es wünschenswert ebenfalls axial verschiebbare zweite Betätigungselemente zum Betätigen des Endeffektors vorzusehen. Die lineare Betätigung des Endeffektors ist hinsichtlich der Handhabbarkeit vorteilhaft. Bei Verwendung bekannter Kupplungseinrichtungen wäre es erforderlich die Linearbewegung der ersten von der Antriebseinrichtung betätigten Betätigungselemente in eine Rotationsbewegung zu überführen, da bekannte Kupplungseinrichtungen Rotationsbewegungen übertragen. Auf der mit dem Manipulatorteil verbundenen Seite der Kupplungseinrichtung müsste sodann die Rotationsbewegung wiederum in eine axiale Bewegung überführt werden. Eine derartige Kupplungseinrichtung in Verbindung mit Umwandeleinrichtungen zur Umwandlung einer Linear- in eine Rotationsbewegung, bzw. zur Umwandlung einer Rotations- in eine Linearbewegung ist äußerst komplex. Eine entsprechende Kupplungseinrichtung wäre nicht nur teuer und aufwändig sondern ist auch stark verlustbehaftet. Dies ist insbesondere bei in der minimal invasiven Chirurgie eingesetzten chirurgischen Manipulationsinstrumenten nicht akzeptabel, da eine sehr exakte und möglichst spielfreie Übertragung von Kräften und Momenten stattfinden muss.

Das erfindungsgemäße chirurgische Manipulationsinstrument, das insbesondere für die minimal invasive Chirurgie geeignet ist, weist eine extrakorporale Antriebseinrichtung sowie einen teilweise intrakorporal anordnenbaren Manipulatorteil auf. Die Antriebseinrichtung weist mehrere axial verschiebbare erste Betätigungselemente auf. Bei den Betätigungselementen handelt es sich beispielsweise um Stangen oder Seile, durch die Axialkräfte übertragen werden. Das Manipulatorteil weist ebenfalls mehrere axial verschiebbare zweite Betätigungselemente auf, bei denen es sich beispielsweise ebenfalls um Stangen und/oder Seile handelt. Die Betätigungselemente dienen zur Betätigung eines Endeffektors.

Die ersten Betätigungselemente sind mit den zweiten Betätigungselementen über eine Kupplungseinrichtung miteinander verbunden. Über die Kupplungseinrichtung erfolgt eine lösbare Verbindung von Betätigungselementen-Paaren. Um eine lösbare Verbindung zu realisieren weist die Kupplungseinrichtung erfindungsgemäß ein erstes Kupplungselement mit einer Hinterschnitt-Ausbuchtung auf, in die ein zweites Kupplungselement eingreift. Durch Zusammenfügen der beiden Kupplungselemente erfolgt somit entsprechend einer Rastverbindung, ein Eingreifen des zweiten Kupplungselementes in die Hinterschnitt-Ausbuchtung. Dies erfolgt vorzugsweise indem die beiden Kupplungselemente axial aufeinander zu geschoben werden. Hierbei weist die Hinterschnitt - Ausbuchtung in axialer Richtung gesehen einen Hinterschnitt auf. Das Verbinden der Betätigungselemente-Paare ist somit durch einfaches Zusammenstecken der Kupplungselemente möglich. Dies hat den Vorteil, dass ein Verbinden der Antriebseinrichtung mit dem Manipulatorteil auf einfache Weise durch Klinikpersonal erfolgen kann. Insbesondere sind keine besonderen Kenntnisse erforderlich.

Erfindungsgemäß ist das erste Kupplungselement rotationssymmetrisch ausgebildet, wobei es bevorzugt ist, dass die Hinterschnitt-Ausbuchtung ringförmig ausgebildet ist. Entsprechend sind die Kupplungsarme in bevorzugter Ausführungsform auf einem Kreisring angeordnet, wobei die Kupplungsarme bei geschlossener Kupplungseinrichtung das erste Kupplungselement umgeben. Bei der erfindungsgemäßen Ausgestaltung des ersten Kupplungselements als rotationssymmetrisches Kupplungselement besteht insbesondere der Vorteil, dass die zu kuppelnden Teile einfach miteinander verbunden werden können, da keine Orientierung der Teile zueinander erforderlich ist.

In bevorzugter Ausführungsform weist das zweite Kupplungselement mehrere verschwenkbare Kupplungsarme auf. Das Verschwenken erfolgt hierbei vorzugsweise durch eine elastische Verformung der Kupplungsarme beim Schließen der Kupplungseinrichtung. Vorzugsweise weisen die Kupplungsarme an ihren freien Enden insbesondere zumindest teilweise radial angeordnete Ansätze auf. Diese Ansätze greifen bei geschlossener Kupplungseinrichtung in die Hinterschnitt-Ausbuchtung ein. Das Schließen der beiden Kupplungselemente erfolgt somit derart, dass die beiden Kupplungselemente axial aufeinander zu bewegt werden, wobei das erste Kupplungselement die Kupplungsarme des zweiten Kupplungselementes beispielsweise nach außen drückt bzw. verschwenkt. Durch ein weiteres Zusammenstecken der Kupplungselemente werden die Kupplungsarme solange verschwenkt, bis sie in ihre Ausgangsstellung zurückschnappen und die Ansätze der Kupplungsarme in die Hinterschnitt-Ausbuchtung eingreifen.

Ferner ist es bevorzugt, dass das erste Kupplungselement steif ausgebildet ist und somit eine hohe Festigkeit aufweist. Insbesondere handelt es sich um einen Vollkörper. Hierdurch ist eine geringe Baugröße möglich.

Um ein möglichst sicheres Verbinden der beiden Kupplungselemente zu gewährleisten ist es bevorzugt, dass das erste Kupplungselement mehrerer insbesondere axial hintereinander angeordnete Hinterschnitt-Ausbuchtungen aufweist. Diese Ausbuchtungen, die in bevorzugter Ausführungsform jeweils ringförmig sind, sind vorzugsweise derart ausgebildet, dass ihr Durchmesser sich stufenförmig vergrößert. Die Kupplungsarme weisen sodann entsprechend mehrerer Ansätze auf, die bei geschlossener Kupplungseinrichtung in die unterschiedlichen Hinterschnitt-Ausbuchtungen eingreifen.

Das erste und/oder das zweite Kupplungselement ist vorzugsweise jeweils mit dem entsprechenden Befestigungselement fest verbunden oder auch einstückig ausgebildet. Hierdurch ist der Platzbedarf verringert.

Vorzugsweise erfolgt das Fixieren des bzw. Halten der Kupplungselemente der Kupplungseinrichtung ausschließlich durch eine Klemmung von außen. Hierdurch kann der Bauraum weiter reduziert werden,

In besonders bevorzugter Ausführungsform ist zusätzlich ein Fixierelement vorgesehen. Das Fixierelement dient zum Halten der Kupplungselemente in geschlossener Stellung. Durch das Fixierelement ist somit ein ungewolltes Öffnen der Kupplungseinrichtung vermieden. Sofern das zweite Kupplungselement Kupplungsarme aufweist, die zum Öffnen oder Schließen nach außen verschwenkt werden, kann ein Fixierelement vorgesehen sein, das die Kupplungsarme in geschlossener Stellung umgibt und somit ein Verschwenken der Kupplungsarme nach außen verhindert. Vorzugsweise ist als Fixierelement eine verschiebbare Hülse vorgesehen. Bei geschlossener Kupplungseinrichtung umgibt die Hülse die beiden Kupplungselemente zumindest teilweise. Insbesondere beim Vorsehen von Kupplungsarmen ist es vorteilhaft eine ringförmige Hülse vorzusehen, die die Kupplungsarme zumindest im Bereich der radial verlaufenden Ansätze umgibt. Zum Öffnen der Kupplungseinrichtung ist es sodann lediglich erforderlich, die Hülse zu verschieben, so dass die beiden Kupplungselemente wieder auseinander gezogen werden können, wobei, sofern das zweite Kupplungselement Kupplungsarme aufweist, diese dann wieder verschwenkbar sind. Vorzugsweise ist das Fixierelement federbelastet wobei die Federbelastung derart vorgesehen ist, dass das Fixierelement entgegen der Federkraft aus der Geschlossenstellung in die Offenstellung überführt werden muss. Hierdurch ist sichergestellt, dass ein Anordnen des Fixierelements in der Geschlossenstellung durch das Bedienpersonal nicht vergessen werden kann.

Bei einer weiteren bevorzugten Ausführungsform der Erfindung weist das zweite Kupplungselement radial verschiebbare Halteelemente wie Kugeln oder dergleichen auf. Durch radiales Verschieben der Halteelemente in die Hinterschnitt-Ausbuchtung erfolgt ein Schließen der Kupplungseinrichtung. Durch ein vorzugsweise vorgesehenes Fixierelement wird vermieden, dass sich die Halteelemente selbsttätig wieder aus der Hinterschnitt-Ausbuchtung heraus bewegen. Dies kann beispielsweise dadurch erfolgen, dass die einzelnen Halteelemente in Richtung der Hinterschnitt-Ausbuchtung federbelastet sind. Auch kann das Fixierelement derart ausgebildet sein, dass es eine Ausnehmung aufweist, in die die Kugeln zum Lösen der Kupplungseinrichtung verschoben werden können und das Fixierelement zum Schließen der Kupplungseinrichtung derart angeordnet ist, dass die Ausnehmung zu den Halteelementen versetzt ist. Hier kann das Fixierelement wiederum verschiebbar ausgebildet sein.

Bei einer bevorzugten Weiterbildung der Erfindung weist das erste Kupplungselement eine axiale Bohrung auf, in die das zweite Kupplungselement oder ein stiftförmiger Ansatz des zweiten Kupplungselements einführbar ist. Bei dieser Ausführungsform ist die Hinterschnitt-Ausbuchtung an einer Innenseite der Bohrung vorgesehen. In die Hinterschnitt-Ausbuchtungen können wiederum Ansätze von Kupplungsarmen oder Halteelemente eingreifen.

In bevorzugter Ausführungsform ist das Fixierelement axial verschiebbar innerhalb des zweiten Kupplungselements angeordnet. Hierbei weist das in dieser Ausführungsform im wesentlichen stiftartig ausgebildete Fixierelement mindestens eine Ausnehmung auf, in die die Halteelemente, bei denen es sich um Kugeln handelt, zum Öffnen der Kupplungseinrichtung gleiten können. Bei dem Verschieben des Fixierelements in die geschlossene Stellung werden die Kugeln durch das Fixierelement in die Hinterschnitt-Ausbuchtungen, die sich in der Innenseite der axialen Bohrung des ersten Kupplungselements befinden, gedrückt.

Gemäß einer weiteren bevorzugten Ausführungsform eines chirurgischen Manipulationsinstruments, die eine unabhängige Erfindung darstellt, kann die Hinterschnitt-Ausbuchtung auch seitlich offen sein. Die Öffnung der Hinterschnitt-Ausbuchtung weist somit zur Längsachse des entsprechenden Betätigungselements einen Winkel von ungleich 0° insbesondere 90° auf. Das zweite Kupplungselement weist bei dieser Weiterbildung einen Ansatz auf, der seitlich in die Hinterschnitt-Ausbuchtung eingeführt wird. Besonders bevorzugt ist es hierbei, dass sämtliche ersten Kupplungselemente eine Hinterschnitt-Ausbuchtung aufweisen, deren Öffnung in dieselbe Richtung weist. Hierdurch ist es möglich die Ansätze der zweiten Kupplungselemente gemeinsam in die Hinterschnitt-Ausbuchtung einzuführen. Insbesondere weisen die zweiten Kupplungselemente Ansätze mit zylindrischem Querschnitt auf, wobei die Hinterschnitt-Ausbuchtungen schlitzähnlich ausgebildet sind und an ihrem Ende eine Rundung aufweisen, die im Wesentlichen dem Radius des zylinderförmigen Ansatz entspricht. Selbstverständlich sind auch andere geometrische Ausgestaltungen möglich, wobei es bevorzugt ist, dass die äußere Form der zweiten Kupplungselemente bzw. der Ansätze der zweiten Kupplungselemente komplementär zu der Ausgestaltung der Hinterschnitt-Ausbuchtung ist.

Bei einer weiteren Ausführungsform sind die Hinterschnitt-Ausbuchtungen ebenfalls seitlich offen und weisen insbesondere einen Öffnungswinkel von 90° zur Längsrichtung der Betätigungselemente auf. Die Öffnungsrichtung der einzelnen Hinterschnitt-Ausbuchtungen liegt hierbei vorzugsweise auf einer Kreislinie. Die zweiten Kupplungselemente weisen wiederum in bevorzugter Ausführungsform Ansätze auf. Das Einführen dieser insbesondere zylindrisch ausgebildeten Ansätze in die Hinterschnitt-Ausbuchtungen erfolgt hierbei durch Verschwenken der Ansätze.

Nachfolgend wird die Erfindung anhand bevorzugter Ausführungsformen unter Bezugnahme auf die anliegenden Zeichnungen näher erläutert.

Es zeigen:
- Figur 1: eine schematische, perspektivische Ansicht eines chirurgischen Manipulationsinstruments
- Figur 2: eine schematische, perspektivische Seitenansicht der Antriebseinrichtung
- Figur 3: eine schematische Schnittansicht eines Teils einer ersten bevorzugten Ausführungsform einer Kupplungseinrichtung
- Figur 4: eine schematische Draufsicht des im Figur 2 dargestellten Bereichs der Kupplungseinrichtung,
- Figur 5: eine schematische Schnittansicht einer weiteren Ausführungsform eines Teils einer Kupplungseinrichtung,
- Figur 6: eine schematische, perspektivische Ansicht einer weiteren Ausführungsform einer Kupplungseinrichtung, die nicht Teil der Erfindung ist, in entkoppeltem Zustand,
- Figur 7: eine schematische, perspektivische Ansicht der in Figur 6 dargestellten Kupplungseinrichtung in eingekuppeltem Zustand,
- Figur 8: eine schematische, perspektivischen Ansicht einer weiteren Ausführungsform einer Kupplungseinrichtung, die nicht Teil der Erfindung ist, in entkoppeltem Zustand und
- Figur 9: eine schematische, perspektivische Ansicht der in Figur 8 dargestellten Kupplungseinrichtung in eingekuppeltem Zustand.

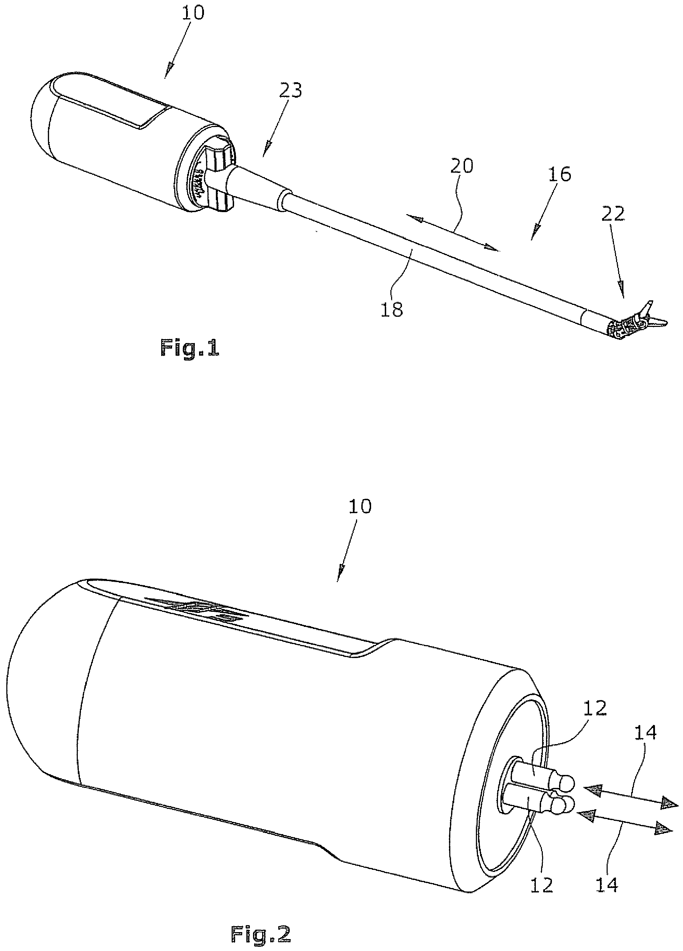

Ein erfindungsgemäßes chirurgisches Manipulationsinstrument, das insbesondere für die minimal invasive Chirurgie geeignet ist weist eine extrakorporale Antriebseinrichtung 10 auf. Diese kann insbesondere einen oder mehrere Elektromotoren und Getriebe aufweisen, wobei hierdurch Betätigungselemente 12 (Figur 2) in axiale Richtung (Pfeile 14) verschoben werden.

Diese ersten Betätigungselemente 12 sind im dargestellten Ausführungsbeispiel als Stangen ausgebildet. Es kann sich jedoch auch um Seile handeln, über die dann ausschließlich Zugkräfte übertragen werden.

Ferner weist das chirurgische Manipulationsinstrument eine Manipulatoreinrichtung 16 (Figur 1) auf. Innerhalb eines rohrförmigen Schafts 18 sind wiederum stabförmige zweite Betätigungselemente angeordnet. Diese sind in Längsrichtung 20 verschiebbar. Die zweiten Betätigungselemente sind mit einem Endeffektor 22 verbunden. Durch Verschieben der zweiten Betätigungselemente erfolgt somit ein Betätigen des Endeffektors. Bei den zweiten Betätigungselementen kann es sich wiederum um stabförmige Betätigungselemente aber auch um Betätigungsseile handeln.

Zur Verbindung der ersten Betätigungselemente 12 und der innerhalb des rohrförmigen Schafts angeordneten zweiten Betätigungselementen ist eine Kupplungseinrichtung 23 vorgesehen.

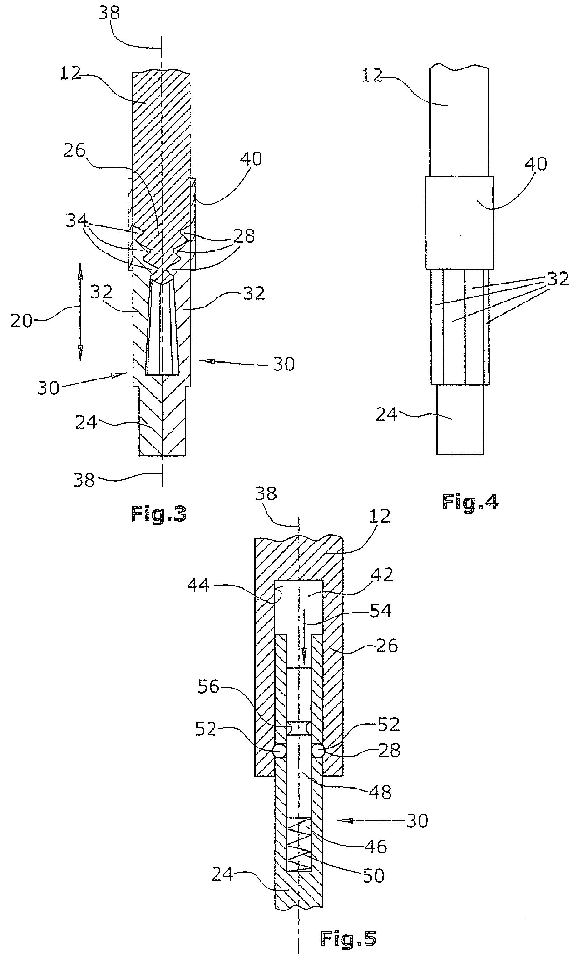

In den in den Figuren 3-5 dargestellten Ausführungsformen sind jeweils Teile der Kupplungseinrichtung dargestellt. Der Einfachheit halber ist hierbei jeweils ein erstes und ein zweites Betätigungselement dargestellt. Üblicherweise weist sowohl die Antriebseinrichtung 10 als auch der Manipulatorteil 16 mindestens zwei, insbesondere mindestens drei derartige Betätigungselemente auf. Diese werden vorzugsweise gleichzeitig ge- oder entkoppelt.

Bei der ersten in den Figuren 3 und 4 dargestellten Ausführungsform der Erfindung ist ein erstes Betätigungselement 12 sowie ein zweites Betätigungselement 24 dargestellt. Das Betätigungselement 12 weist am Ende einen ein erstes Kupplungselement 26 ausbildenden kegelstumpfförmigen Ansatz aus. Dieser weist im dargestellten Ausführungsbeispiel drei jeweils ringförmig ausgebildete Hinterschnitt-Ausbuchtungen 28 auf. Durch die dargestellte Ausbuchtung 28 ist in axialer Richtung bzw. in Längsrichtung der Betätigungselemente 12 und 24 ein Hinterschnitt realisiert.

Das zweite Betätigungselement 24 weist an dem in Richtung des ersten Betätigungselements 12 weisenden Ende ein zweites Kupplungselement 30 auf. Das Kupplungselement 30 umfasst im dargestellten Ausführungsbeispiel mehrere Kupplungsarme 32. Die Kupplungsarme 32 weisen an ihrem freien Ende radiale im dargestellten Ausführungsbeispiel nach innen gerichtete Ansätze 34 auf. Bei geschlossener Kupplungseinrichtung wie sie in Figur 3 dargestellt ist greifen die Ansätze 34 in die Hinterschnitt-Ausbuchtungen 28 ein. Dies ist dadurch möglich, dass die Kupplungsarme elastisch ausgebildet sind und im wesentlichen jeweils um einen Punkt 36 elastisch schwenkbar sind. Dies hat zur Folge, dass beim Zusammenstecken der beiden Kupplungselemente 26,30 die Kupplungsarme 32 nach außen gedrückt werden bis sich die beiden Kupplungselemente in der Geschlossenstellung befinden und die Ansätze 34 in die Hinterschnitt-Ausbuchtung 28 einrasten.

Im dargestellten Ausführungsbeispiel sind mehrere ringförmige Hinterschnitt-Ausbuchtungen 28 vorgesehen. Diese weisen in Figur 3 von unten nach oben betrachtet jeweils einen größeren Durchmesser auf und sind insofern stufenförmig angeordnet. Das erste Kupplungselement 26 sowie das im dargestellten Ausführungsbeispiel einstückig mit dem Kupplungselement ausgebildete erste Betätigungselement 12 sind zu einer Längsachse 38 rotationssymmetrisch ausgebildet.

Das zweite Kupplungselement 30 weist im dargestellten Ausführungsbeispiel mehrere auf einer Kreislinie angeordnete Kupplungsarme 32 auf. Diese sind im dargestellten Ausführungsbeispiel wiederum einstückig mit dem zweiten Betätigungselement 24 ausgebildet. Das Betätigungselement 24 ist zu der Längsachse 38 ebenfalls rotationssymmetrisch.

Um sicherzustellen, dass die Kupplungsarme 32 in der Geschlossenstellung verbleiben, ist ein Fixierelement 40 vorgesehen. Im dargestellten Ausführungsbeispiel handelt es sich bei dem Fixierelement um eine in Längsrichtung 20 verschiebbare Hülse. Die Hülse 40 umgibt in geschlossenem Zustand der Kupplungseinrichtung die beiden Kupplungselemente 26,30. Da die Kupplungsarme 32 zum Öffnen der Kupplung nach außen verschwenkt werden müssten, ist durch die Hülse 40 ein ungewolltes Öffnen der Kupplungseinrichtung vermieden. Zum Öffnen der Kupplungseinrichtung müsste die Hülse 40 in Figur 3 nach oben verschoben werden. In dieser Stellung können die beiden Kupplungselemente 26,30 sodann in axiale Richtung auseinander gezogen werden. Da üblicherweise zwei oder mehr Betätigungselement-Paare 12-24 insbesondere gleichzeitig miteinander verbunden werden ist es bevorzugt, dass die Hülsen 40 im Betrieb zueinander verschieblich, aber zu den Antriebselementen 12-24 ortsfest sind.

Ebenso ist es möglich, dass das erste Kupplungselement beispielsweise kugelförmig ausgebildet und fest mit dem ersten Betätigungselement 12 verbunden ist. Auch in einer derartigen Kugel ist eine Hinterschnitt-Ausbuchtung ausgebildet in die entsprechende radiale Ansätze von Kupplungsarmen eingreifen können.

Bei dem weiteren in Figur 5 dargestellten Ausführungsbeispiel der Erfindung sind identische Bauteile mit dem selben Bezugszeichen gekennzeichnet.

In diesem Ausführungsbeispiel ist das erste Kupplungselement 26 wiederum rotationssymmetrisch zur Längsachse 38 ausgebildet. Das im wesentlichen zylindrisch ausgebildete erste Kupplungselement 26 weist eine mittige, zentrale sich in axialer Richtung erstreckende Bohrung 42 auf. An einer Innenseite 44 der Bohrung 42 ist die wiederum ringförmig ausgebildete Hinterschnitt-Ausbuchtung 28 angeordnet.

Das zweite Kupplungselement 30 ist in dem dargestellten Ausführungsbeispiel stabförmig ausgebildet und zum Schließen der Kupplungseinrichtung wie in Figur 5 dargestellt in die axiale Bohrung 42 eingeführt. Das zweite Kupplungselement 30 weist ebenfalls eine zentrale in axiale Richtung verlaufende Bohrung 46 auf. In dieser ist ein in diesem Ausführungsbeispiel stabförmig ausgebildetes Fixierelement 48 angeordnet, wobei das stabförmige Fixierelement 48 durch eine Feder 50 stets in Richtung des ersten Betätigungselements 12 gedrückt wird. In dieser Stellung liegen im dargestellten Ausführungsbeispiel als Kugeln ausgebildete Halteelemente 52 an der glatten, zylindrischen Außenfläche des Fixierelements 48 an. Dies führt dazu, dass die Kugeln 52 in Figur 5 radial nach außen in die Hinterschnitt-Ausbuchtung 28 gedrückt werden. Hierdurch befindet sich das Kupplungselement in einer Geschlossenstellung. Auf Grund der Federbelastung des Fixierelements 48 ist ein selbsttätiges ungewolltes Öffnen vermieden. Zum Öffnen der Kupplungseinrichtung ist es erforderlich das Fixierelement 48 in Richtung eines Pfeils 54, d.h. in Richtung des zweiten Betätigungselements 24 zu verschieben. Dies kann beispielsweise über einen nicht dargestellten stiftförmigen Ansatz erfolgen, der durch eine schlitzförmige Öffnung in dem Kupplungselement 30 nach außen geführt ist. Durch Verschieben dieses Ansatzes erfolgt sodann ebenfalls ein Verschieben des Fixierelements 48. Da bei dieser Ausführungsform vorzugsweise wiederum mindestens zwei Betätigungselemente-Paare 12-24 vorgesehen sind, ist es wiederum bevorzugt, dass die Fixierelemente 48 gemeinsam betätigbar sind.

Durch das Verschieben des Fixierelements 48 in Richtung des Pfeils 54 erfolgt eine Verschiebung der Ausnehmung 56 auf Höhe der Kugeln 52. Hierdurch ist es möglich das zweite Betätigungselement 24 aus der axialen Bohrung 52 herauszuziehen, da die Kugeln 52 radial nach innen verschiebbar sind.

Selbstverständlich ist es auch möglich die beiden vorstehend beschriebenen Ausführungsformen miteinander zu kombinieren, insbesondere einzelne Betätigungselemente-Paare wie anhand Figur 3,4 und andere wie anhand Figur 5 beschriebene miteinander zu koppeln.

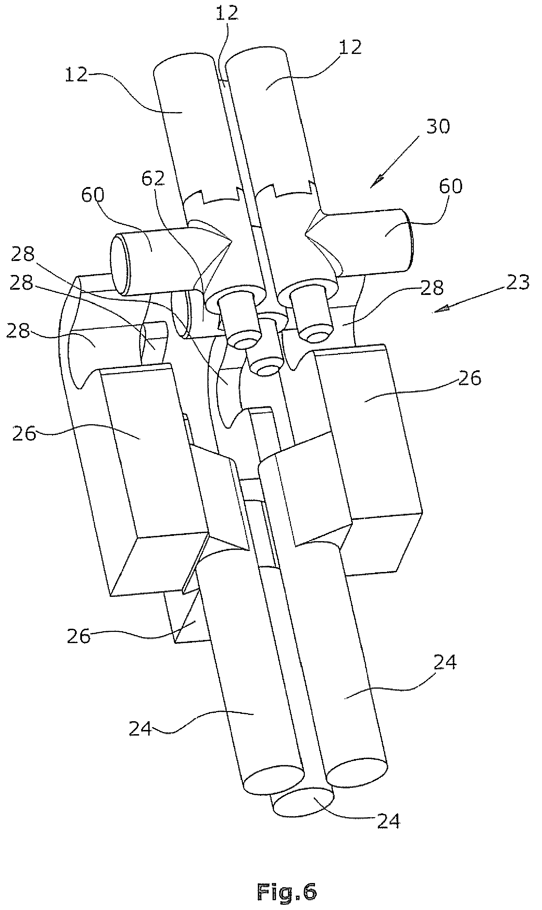

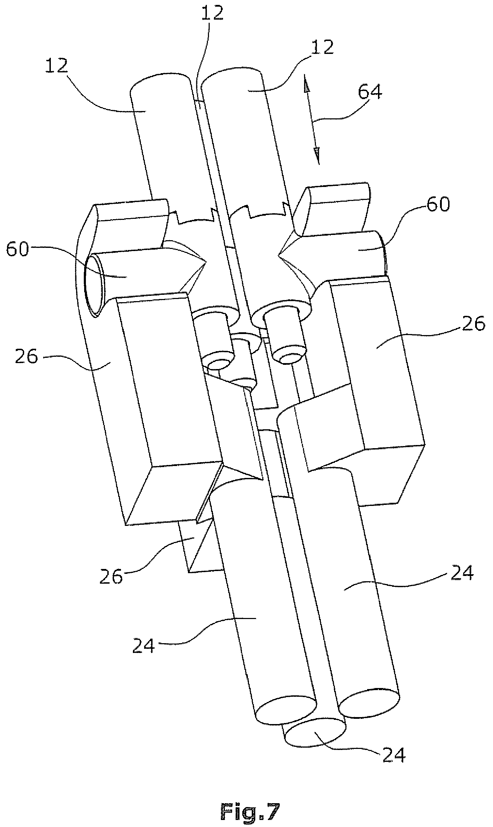

Bei einer weiteren Ausführungsform (Figur 6 und 7) handelt es sich um eine selbstständige Erfindung der Kupplungseinrichtung. Die Kupplungseinrichtung dient ebenfalls zum Verbinden von ersten Betätigungseinrichtungen 12, bei denen es sich im dargestellten Ausführungsbeispiel um Stäbe handelt, mit zweiten Betätigungseinrichtungen 24, bei denen es sich ebenfalls um stabförmige Betätigungseinrichtungen handelt. Die Betätigungseinrichtungen 12, 24 sind, wie anhand der vorstehend beschriebenen Ausführungsformen erläutert, mit einer Antriebseinrichtung 10 (Figur 1) bzw. einem Endeffektor 22 verbunden.

Mit den zweiten Betätigungseinrichtungen 24 sind zur Ausgestaltung der Kupplungseinrichtung 23 erste Kupplungselemente 26 verbunden. Diese weisen jeweils eine Hinterschnitt-Ausbuchtung 28 auf. Bei dem dargestellten Ausführungsbeispiel weisen sämtliche Hinterschnitt-Ausbuchtungen 28 in dieselbe Richtung. Ferner weisen die Hinterschnitt-Ausbuchtungen 28 zu den Längsachsen der zweiten Betätigungseinrichtungen 24 einen Winkel von 90° auf, so dass die Hinterschnitt-Ausbuchtungen 28 seitlich offen sind.

Die mit den ersten Betätigungseinrichtungen 12 verbundenen ersten Kupplungselemente 30 weisen einen im dargestellten Ausführungsbeispiel zylindrischen Zapfen 60 auf. Die beiden in Figur 6 vorderen Zapfen 60 weisen in entgegengesetzte Richtung. Um ein gemeinsames Einführen der Zapfen 60 in die Hinterschnitt-Ausbuchtungen 28 zu ermöglichen, ragt der Zapfen 62 des hinteren Betätigungselements 12 in beide Richtungen und greift in die beiden Teile 28 der hinteren Hinterschnitt-Ausbuchtung. Der Zapfen 62 wird beim Einführen in die geteilte Hinterschnitt-Ausbuchtung 28 zwischen den beiden ersten Kupplungselementen 26 hindurchgeführt.

In eingeführtem Zustand (Figur 7) können von den ersten Betätigungseinrichtungen 12 Axialkräfte in Richtung eines Pfeils 64, d.h. in Richtung der Längsachsen der ersten Betätigungseinrichtungen 12, auf die zweiten Betätigungseinrichtungen 24 übertragen werden.

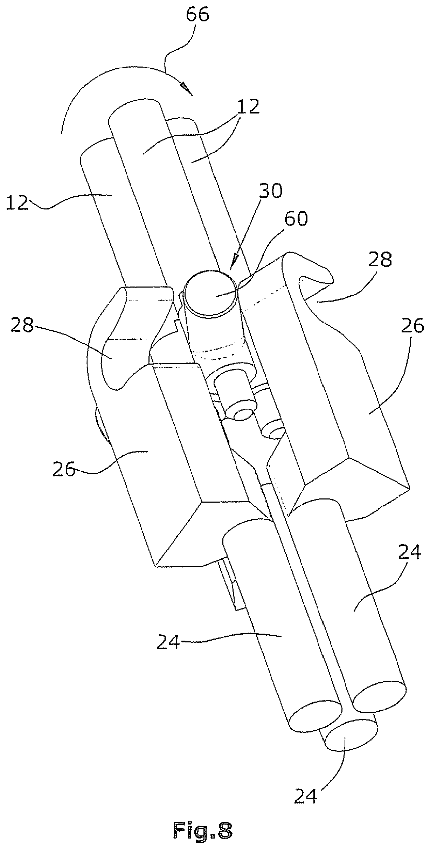

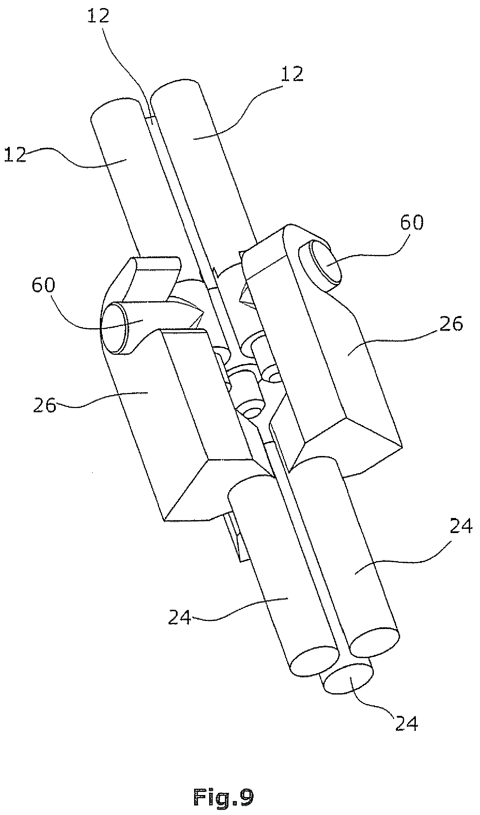

Bei einer weiteren Ausführungsform einer weiteren Kupplungseinrichtung 23, die ebenfalls eine selbstständige Erfindung darstellt (Figur 8 und 9), handelt es sich um eine der in den Figuren 6 und 7 dargestellten Ausführungsform ähnlichen Ausführungsform. Der wesentliche Unterschied besteht darin, dass das Einkuppeln durch gemeinsames Verdrehen der ersten Betätigungselemente 12 in Richtung eines Pfeils 66 (Figur 8) erfolgt. Dementsprechend sind die Hinterschnitt-Ausbuchtungen 28 der zweiten Kupplungselemente 26 auf einer Kreislinie angeordnet und in tangentiale Richtung offen. In diesem Ausführungsbeispiel mit drei ersten Betätigungseinrichtungen 12 weisen diese jeweils radial nach außen verlaufende zylindrische Ansätze 60 auf. Diese weisen bezogen auf die Längsachse der jeweiligen ersten Betätigungseinrichtungen Winkel von 90° auf. Untereinander weisen die Zapfen einen Winkel von 120° auf. Die drei Betätigungselemente 12 einschließlich der ersten Kupplungselemente 30 sind in diesem Ausführungsbeispiel identisch ausgebildet. Das gilt auch für die zweiten Betätigungselement 24 in Verbindung mit den ersten Kupplungselementen 26.

## Patentansprüche

1. Chirurgisches Manipulationsinstrument, insbesondere für die minimal invasive Chirurgie, mit
einer extrakorporalen Antriebseinrichtung (10), mit mehreren axial verschiebbaren ersten Betätigungselementen (12),
einem teilweise intrakorporalen Manipulatorteil (16), mit mehreren axial verschiebbaren zweiten Betätigungselementen (24) zum Betätigen eines Endeffektors (22) und
einer Kupplungseinrichtung (23) zum lösbaren Verbinden von je einem Betätigungselementen-Paar (12,24), wobei die Kupplungseinrichtung (23) ein erstes Kupplungslement (26) mit einer Hinterschnitt-Ausbuchtung (28) aufweist, in die ein zweites Kupplungselement (30) eingreift,
**dadurch gekennzeichnet, dass**
das erste Kuppelelement (26) insbesondere um seine Längsachse (38), die mit der Längsachse des ersten Betätigungselements (12) zusammenfällt, rotationssymmetrisch ist.

2. Chirurgisches Manipulationsinstrument nach Anspruch 1, **dadurch gekennzeichnet, dass** das zweite Kupplungselement (30) schwenkbare Kupplungsarme (32) aufweist, die an ihrem freien Ende einen insbesondere radial angeordneten Ansatz (34) aufweisen, der bei geschlossener Kupplungseinrichtung in die Hinterschnitt-Ausbuchtung (28) eingreift.

3. Chirurgisches Manipulationsinstrument nach Anspruch 2, **dadurch gekennzeichnet, dass** die Kupplungsarme (32) auf einem Kreisring angeordnet sind und bei geschlossener Kupplungseinrichtung das erste Kupplungselement (26) umgeben.

4. Chirurgisches Manipulationsinstrument, nach einem der Ansprüche 1-3, **dadurch gekennzeichnet, dass** die Hinterschnitt-Ausbuchtung (28) ringförmig ausgebildet ist.

5. Chirurgisches Manipulationsinstrument, nach einem der Ansprüche 1-4, **dadurch gekennzeichnet, dass** das erste Kupplungselement (26) mehrere insbesondere axial hintereinander angeordnete Hinterschnitt-Ausbuchtungen (28) aufweist.

6. Chirurgisches Manipulationsinstrument, nach Anspruch 4 oder 5, **dadurch gekennzeichnet, dass** sich der Durchmesser der ringförmig ausgebildeten Hinterschnitt-Ausbuchtungen (28) in axialer Richtung stufenweise vergrößert.

7. Chirurgisches Manipulationsinstrument, nach einem der Ansprüche 1-6 **dadurch gekennzeichnet, dass** das erste und oder das zweite Kuppelelement (26,30) mit dem ersten bzw. zweiten Betätigungselement (12,24) fest verbunden, insbesondere einstückig ausgebildet ist.

8. Chirurgisches Manipulationsinstrument, nach einem der Ansprüche 1-7, **gekennzeichnet durch** ein Fixierelement (40,48) zum Halten der Kuppelelemente (26,30) in Geschlossenstellung, um ein Öffnen der Kupplungseinrichtung (23) zu vermeiden.

9. Chirurgisches Manipulationsinstrument, nach Anspruch 8, **dadurch gekennzeichnet, dass** das Fixierelement (40) die Kuppelelemente (26,30) in Geschlossenstellung zumindest teilweise umgibt, wobei das Fixierelement (40) vorzugsweise eine in axiale Richtung verschiebbare Hülse aufweist.

10. Chirurgisches Manipulationsinstrument, nach einem der Ansprüche 1-9, **dadurch gekennzeichnet, dass** das zweite Kuppelelement (30) zum Schließen der Kuppeleinrichtung (23) radial in die Hinterschnitt-Ausbuchtung (28) verschiebbare Halteelemente (52) aufweist.

11. Chirurgisches Manipulationsinstrument, nach einem der Ansprüche 1-10, **dadurch gekennzeichnet, dass** das erste Kuppelelement (26) eine axiale Bohrung (42) aufweist, in die das das zweite Kuppelelement (30) einführbar ist, wobei die Hinterschnitt-Ausbuchtung (28) an einer Innenseite (44) der Bohrung (42) vorgesehen ist.

12. Chirurgisches Manipulationsinstrument, nach Anspruch 10 oder 11, **dadurch gekennzeichnet, dass** das axial verschiebbare Fixierelement (48) in der Geschlossenstellung die Halteelemente (52) radial nach außen drückt und eine Ausnehmung (56) aufweist in die die Halteelemente (52) zum Öffnen gleiten.

## Claims

1. A surgical manipulation instrument, in particular for minimally invasive surgery, comprising:
an extra-corporeal drive device (10) comprising a plurality of axially displaceable first actuation elements (12),
a partially intra-corporeal manipulator part (16) comprising a plurality of axially displaceable second actuation means (24) for actuating an end effector (22), and
a coupling device (23) for a detachable coupling of a respective actuation elements pair (12, 24), said coupling device (23) comprising a first coupling element (26) with an undercut recess (28) into which a second coupling element (30) engages,
**characterized in that**
the first coupling element (26) is rotationally symmetric in particular with respect to its longitudinal axis (38) that coincides with the longitudinal axis of the first actuation element (12).

2. The surgical manipulation instrument of claim 1, **characterized in that** the second coupling element (30) comprises pivotable coupling arms (32) having their free end provided with a projection (34), in particular a radially arranged projection, which engages into the undercut recess (28) when the coupling device is in the closed state.

3. The surgical manipulation instrument of claim 2, **characterized in that** the coupling arms (32) are arranged on a circular ring and surround the first coupling element (26) when the coupling device is in the closed state.

4. The surgical manipulation instrument of one of claims 1-3, **characterized in that** the undercut recess (28) is annular in shape.

5. The surgical manipulation instrument of one of claims 1-4, **characterized in that** the first coupling element (26) comprises a plurality of undercut recesses (28) arranged in particular one after the other in the axial direction.

6. The surgical manipulation instrument of claim 4 or 5, **characterized in that** the diameter of the annularly shaped undercut recesses (28) increases step-wise in the axial direction.

7. The surgical manipulation instrument of one of claims 1-6, **characterized in that** the first and/or the second coupling element (26, 30) is rigidly connected, in particular formed integrally, with the first or the second actuation element (12, 24), respectively.

8. The surgical manipulation instrument of one of claims 1-7, **characterized by** a fixing element (40, 48) for holding the coupling elements (26, 30) in the closed position in order to prevent the coupling device (23) from being opened.

9. The surgical manipulation instrument of claim 8,'**characterized in that** the fixing element (40) at least partly surrounds the coupling elements (26, 30) in the closed position, the fixing element (40) preferably comprising an axially displaceable sleeve.

10. The surgical manipulation instrument of one of claims 1-9, **characterized in that** the second coupling element (30) comprises holding elements (52), adapted to be displaced radially into the undercut recess (28), for closing the coupling device (23).

11. The surgical manipulation instrument of one of claims 1-10, **characterized in that** the first coupling element (26) comprises an axial bore (42) into which the second coupling element (30) may be inserted, the undercut recess (28) being provided on an inner side (44) of the bore (42).

12. The surgical manipulation instrument of claim 10 or 11, **characterized in that**, in the closed position, the axially displaceable fixing element (48) presses the holding elements (52) radially outward and comprises a recess (56) into which the holding elements (52) slide for opening.

## Revendications

1. Instrument de manipulation à usage chirurgical se prêtant notamment à la chirurgie mini-invasive, comprenant un dispositif d'entraînement extracorporel (10) comportant plusieurs premiers éléments d'actionnement (12) à déplacement axial,
un élément manipulateur partiellement intracorporel (16) comportant plusieurs seconds éléments d'actionnement (24) à déplacement axial, qui servent à actionner un effecteur terminal (22), et
un dispositif d'accouplement (23) pour la liaison amovible de respectivement une paire d'éléments d'actionnement (12, 24), le dispositif d'accouplement (23) présentant un premier élément d'accouplement (26) pourvu d'une courbure à contre-dépouille (28) dans laquelle s'engage un second élément d'accouplement (30), **caractérisé en ce que**
le premier élément d'accouplement (26) est symétrique en rotation, en particulier autour de son axe longitudinal (38) qui coïncide avec l'axe longitudinal du premier élément d'actionnement (12).

2. Instrument de manipulation à usage chirurgical selon la revendication 1, **caractérisé en ce que** le second élément d'accouplement (30) présente des bras d'accouplement (32) pivotants, qui comportent à leur extrémité libre un épaulement (34) disposé en particulier radialement, qui s'engage dans la courbure à contre-dépouille (28) lorsque le dispositif d'accouplement est en position fermée.

3. Instrument de manipulation à usage chirurgical selon la revendication 2, **caractérisé en ce que** les bras d'accouplement (32) sont disposés sur un anneau circulaire et entourent le premier élément d'accouplement (26) lorsque le dispositif d'accouplement est en position fermée.

4. Instrument de manipulation à usage chirurgical selon l'une des revendications 1 à 3, **caractérisé en ce que** la courbure à contre-dépouille (28) est de forme circulaire.

5. Instrument de manipulation à usage chirurgical selon l'une des revendications 1 à 4, **caractérisé en ce que** le premier élément d'accouplement (26) présente plusieurs courbures à contre-dépouille (28) disposées les unes derrière les autres, en particulier en direction axiale.

6. Instrument de manipulation à usage chirurgical selon la revendication 4 ou 5, **caractérisé en ce que** le diamètre des courbures à contre-dépouille (28) de forme circulaire augmente progressivement dans la direction axiale.

7. Instrument de manipulation à usage chirurgical selon l'une des revendications 1 à 6, **caractérisé en ce que** le premier et/ou le second élément d'accouplement (26, 30) est solidaire, en particulier formé d'un seul tenant, avec le premier ou le second élément d'actionnement (12, 24).

8. Instrument de manipulation à usage chirurgical selon l'une des revendications 1 à 7, **caractérisé par** un élément de fixation (40, 48) destiné à maintenir les éléments d'accouplement (26, 30) en position fermée afin d'éviter l'ouverture du dispositif d'accouplement (23).

9. Instrument de manipulation à usage chirurgical selon la revendication 8, **caractérisé en ce que** l'élément de fixation (40) entoure au moins partiellement les éléments d'accouplement (26, 30) en position fermée, l'élément de fixation (40) présentant de préférence un manchon déplaçable en direction axiale.

10. Instrument de manipulation à usage chirurgical selon l'une des revendications 1 à 9, **caractérisé en ce que** le second élément d'accouplement (30) présente, pour la fermeture du dispositif d'accouplement (23), des éléments de retenue (52) déplaçables radialement dans la courbure à contre-dépouille (28).

11. Instrument de manipulation à usage chirurgical selon l'une des revendications 1 à 10, **caractérisé en ce que** le premier élément d'accouplement (26) présente un alésage axial (42) dans lequel peut être introduit le second élément d'accouplement (30), la courbure à contre-dépouille (28) étant prévue sur une face intérieure (44) de l'alésage (42).

12. Instrument de manipulation à usage chirurgical selon la revendication 10 ou 11, **caractérisé en ce que** l'élément de fixation (48) à déplacement axial comprime les éléments de retenue (52) radialement vers l'extérieur en position fermée, et présente un évidement (56) dans lequel glissent les éléments de retenue (52) pour l'ouverture.
